# EUROPÄISCHE PATENTSCHRIFT

(11) **EP 1 018 317 B1**
(45) Veröffentlichungstag und Bekanntmachung des Hinweises auf die Patenterteilung: **05.10.2005**
(21) Anmeldenummer: 99123862.7
(22) Anmeldetag: 01.12.1999
(51) Int. Cl.: A61B 17/72

(54) **System zur Positionierung eines intramedulären Kraftträgers, insbesondere Marknagels**
System for positioning an intramedullary load carrier, particularly a nail
Système pour le positionnement d'un élément intramédullaire de transfert de charge, en particulier un clou

(30) Priorität: 05.01.1999 DE 19900133
(43) Veröffentlichungstag der Anmeldung: 12.07.2000
(73) Patentinhaber: Intraplant AG, CH-6330 Cham (CH)
(72) Erfinder: Friedl, Wilhelm, Prof. Dr. med., 63808 Haibach (DE)
(74) Vertreter: Popp, Eugen

(56) Entgegenhaltungen:
- WO-A-94/12126
- WO-A-94/27508
- US-A- 4 697 585

## Beschreibung

Die Erfindung betrifft ein System bestehend aus einem intramedulären Kraftträger, insbesondere Marknagel, und wenigstens einem diesem zugeordneten Knochen-Verriegelungselement.

Derartige Kraftträger werden zur Heilung von Frakturen röhrenartiger Knochen verwendet. Dazu gehören auch Systeme zur Versorgung subtrochanterer und pertrochanterer Frakturen sowie Schenkelhalsfrakturen, die jeweils einen von proximal in den Markraum eines Femur einführbaren Verriegelungsnagel, der einen distalen Bereich mit mindestens einer Querbohrung für die Aufnahme eines distalen Verriegelungselements sowie einen proximalen Abschnitt mit einer schrägen Durchgangsöffnung aufweist, und einen von lateral durch die schräge Durchgangsöffnung hindurch in den Schenkelhals und Femurkopf einführbaren Schenkelhalsteil, z.B. Schenkelhalsschraube oder Schenkelhalsklinge umfassen, wobei in das proximale Ende des Verriegelungsnagels ein Verriegelungsstift oder dergleichen einsetzbar sein kann, mittels dem die axiale Bewegung des Schenkelhalsteils, d.h. der Schenkelhalsschraube oder Schenkelhalsklinge innerhalb der schrägen Durchgangsöffnung im proximalen Abschnitt des Verriegelungsnagels entweder begrenzbar oder vollständig blockierbar ist. Bezüglich einer vorteilhaften Konstruktion letztgenannten Systems wird auf die auf die Rechtsvorgängerin der Anmelderin zurückgehende WO 94/27508 verwiesen. Dort ist eine Konstruktion dargestellt, die dadurch gekennzeichnet ist, daß der Schenkelhalsteil als Schenkelhalsklinge mit einem von einem kreis- bzw. rotationssymmetrischen Querschnitt abweichenden Querschnitt, insbesondere Rechteck-Querschnitt, ausgebildet ist, wobei die schräge Durchgangsöffnung für die Aufnahme der Schenkelhalsklinge einen komplementären, d.h. ebenfalls insbesondere Rechteck-Querschnitt aufweist derart, daß die Schenkelhalsklinge dauerhaft spielfrei und rotationsstabil innerhalb der schrägen Durchgangsöffnung gehalten ist.

Zur Versorgung von Schenkelhalsfrakturen hat sich die Konstruktion bzw. das System nach der WO 94/27508 bestens bewährt, auf dem der Oberbegriff des Anspruchs 1 basiert. Es handelt sich um ein rotationsstabiles System, und zwar sowohl in Relation zwischen Verriegelungsnagel und Schenkelhalsklinge einerseits als auch in Relation zwischen Schenkelhalsklinge und Schenkelhalsknochen andererseits.

Ziel der vorliegenden Erfindung ist es, ein System der eingangs genannten Art zu schaffen, welches sowohl die vorgenannten Vorteile des bekannten Systems nach der WO 94/27508 aufweist als auch geeignet ist für kleinere Dimensionen, die einen Durchtritt eines Verriegelungselements durch den intramedulären Kraftträger bzw. Marknagel hindurch aus Festigkeitsgründen nicht mehr erlauben, so daß das System nicht nur zur Versorgung von Schenkelhalsfrakturen, sondern auch zur Versorgung von Frakturen im Bereich des proximalen Humerus, der proximalen Tibia oder des distalen Femur eingesetzt werden kann.

Diese Aufgabe wird erfindungsgemäß dadurch gelöst, daß das Verriegelungselement wenigstens zwei Zinken aufweist, zwischen denen der intrameduläre Kraftträger form- und/oder kraftschlüssig gehalten ist. Der Kern der vorliegenden Erfindung liegt also darin, daß ein Verriegelungselement bereitgestellt wird, welches den zugeordneten Marknagel rotationsstabil hält, ohne diesen zu durchdringen, so daß letzterer anpassungsgerecht schlank bzw. dünn ausgebildet werden kann. Gleichzeitig ist durch die Ausbildung von wenigsten zwei Zinken auch eine Rotationsstabilität zwischen Verriegelungselement und zugeordnetem Marknagel einerseits und Knochenfraktur bzw. Frakturelementen andererseits gewährleistet. Das erfindungsgemäße Nagelsystem eignet sich also besonders gut bei kleinen Nageldurchmessern, wie sie z.B. für die Versorgung des Humerus erforderlich sind. Vorzugsweise ist das Verriegelungselement nach Art einer Stimmgabel ausgebildet, wobei die Zinken jeweils einen I-förmigen Querschnitt aufweisen. Es ist jedoch auch jeder andere Zinken-Querschnitt denkbar, da die Rotationsstabilität relativ zum Knochen schon allein dadurch erreicht wird, daß das Verriegelungselement zwei im Abstand voneinander sich etwa parallel zueinander erstreckende Zinken aufweist. Dementsprechend können die Zinken auch mit einem T-, Stern-, Rechteck-, Dreieck-, Kreis-, Oval- oder dergleichen Querschnitt ausgebildet sein. Die I-förmige Querschnittsform der Zinken ist jedoch besonders vorteilhaft, da auch bei dünnwandiger Ausbildung in sich sehr rotationsstabil und bruchsicher. Das freie Ende der Zinken ist vorzugsweise mit einer Schneide oder Spitze versehen, um das Einschlagen des Verriegelungselements in den Knochen zu erleichtern.

Die Zinken und/oder das Verbindungselement derselben können eine sich in Zinken-Längsrichtung erstreckende Bohrung für einen Führungs- bzw. Zieldraht aufweisen.

Das erfindungsgemäße System kann sowohl im proximalen als auch distalen Endbereich des intramedulären Kraftträgers vorgesehen sein.

Um eine gewünschte Zuordnung zwischen Kraftträger und Verriegelungselement sicherzustellen, kann der Kraftträger am proximalen und/oder distalen Endbereich wenigstens eine sich quer zur Längsachse erstreckende Ausnehmung zur formschlüssigen Aufnahme wenigstens einer Zinke des Verriegelungselements aufweisen.

Eine Ausführungsform des erfindungsgemäßen Systems ist dadurch gekennzeichnet, daß der Kraftträger am proximalen und/oder distalen Endbereich zur formschlüssigen Anlage wenigstens einer an der dem Kraftträger zugewandten Seite abgeflachten Zinke des Verriegelungselements wenigstens an der dieser Zinke zugewandten Seite entsprechend abgeflacht ist.

Eine alternative Ausführungsform ist dadurch gekennzeichnet, daß die Zinken des Verriegelungselements einen Kraftträger-Ver riegelungsabschnitt und einen Knochen-Verankerungsabschnitt aufweisen, wobei im Bereich des ersteren die Zinken an der dem Kraftträger zugewandten Seite profiliert sind entsprechend einer komplementären Profilierung der den Zinken zugeordneten Aufnahmen des Kraftträgers.

Das Verriegelungselement kann drei oder mehr Zinken umfassen, die vorzugsweise in Kraftträger-Längsrichtung jeweils versetzt zueinander angeordnet sind.

Wie bereits oben erwähnt, können die Zinken im Bereich des Knochen-Verankerungsabschnitts jeweils nach Art einer Klinge mit I-, T-, Rechteck- oder Oval-Querschnitt oder stiftartig mit Stern-, Dreieck- oder Kreis-Querschnitt ausgebildet sein.

Die Ausnehmungen zur Aufnahme wenigstens einer Zinke des Verriegelungselements am proximalen und/oder distalen Endbereich des Kraftträgers sind in Längsrichtung desselben vorzugsweise verlängert, so daß sowohl eine statische als auch dynamische Verriegelung des Kraftträgers durch das Verriegelungselement möglich ist. Alternativ kann zu diesem Zweck am distalen Ende eines Marknagels eine Langloch-Querbohrung vorgesehen sein, durch die hindurch ein distaler Verriegelungsbolzen oder eine distale Verriegelungsschraube zur Verankerung im Knochen hindurchführbar ist, wobei der Verriegelungsbolzen bzw. die Verriegelungsschraube zur statischen distalen Verriegelung des Nagels am proximalen Ende und zur dynamischen distalen Verriegelung des Nagels am distalen Ende der Langloch-Querbohrung eingeführt wird.

Am proximalen Einschlagende kann der Nagel eine Einrichtung aufweisen, mittels der ein Zurückziehen des Nagels nach Verriegelung im distalen Bereich möglich ist. Auf diese Weise läßt sich eine Frakturkompression vor proximaler Verriegelung des Nagels mittels des erfindungsgemäßen Verriegelungselements herstellen. Bei dieser Kompressionseinrichtung handelt es sich um eine an sich bekannte Konstruktion, die unter Fachkreisen als UHN (Unreamed Humerus Nagel)-System bezeichnet wird.

Eine Verriegelung des Nagels im distalen Bereich, d.h. im Bereich nahe der Nagelspitze mittels eines Verriegelungselements der hier vorgeschlagenen Art ist immer dann von Bedeutung, wenn die Nagelspitze in einem spongiosen Bereich zu liegen kommt, z.B. wenn der Nagel retrograd am Humerus eingesetzt wird und mit der Spitze somit im Humeruskopf zu liegen kommt. Auch für sehr weit körperfern gelegene metaphysäre Frakturen des Unterschenkels ist eine Verriegelung gemäß Erfindung am distalen Ende des Nagels von Vorteil.

Zur Versorgung von Schenkelhalsfrakturen erstrecken sich die Ausnehmungen zur Aufnahme wenigstens einer Zinke des Verriegelungselements schräg zur Längsachse des Kraftträgers entsprechend der Erstreckung einer herkömmlichen Schenkelhalsschraube oder Schenkelhalsklinge.

Nachstehend werden vorteilhafte Ausführungsformen der Erfindung anhand der beigefügten Zeichnung erläutert. Es zeigen:
- Fig. 1: ein erfindungsgemäßes System zur Versorgung von Schenkelhalsfrakturen in Vorderansicht;
- Fig. 2: das System gemäß Fig. 1 teilweise im Längsschnitt, teilweise in Lateralansicht;
- Fig. 3: das Verriegelungselement des Systems gemäß den Fig. 1 und 2 in Draufsicht;
- Fig. 4: das Verriegelungselement gemäß Fig. 3 in Stirnan- sicht;
- Fig. 5: einen Teil einer alternativen Ausführungsform eines erfindungsgemäßen Systems mit einem Verriegelungsele- ment, welches drei relativ zueinander versetzt ange- ordnete Haltezinken aufweist, in Lateralansicht; und
- Fig. 6: ein Beispiel für die Verriegelung eines Marknagels im distalen Bereich bzw. im Bereich nahe der Nagelspitze mittels eines erfindungsgemäßen Verriegelungselements teilweise im Schnitt, teilweise in Lateralansicht.

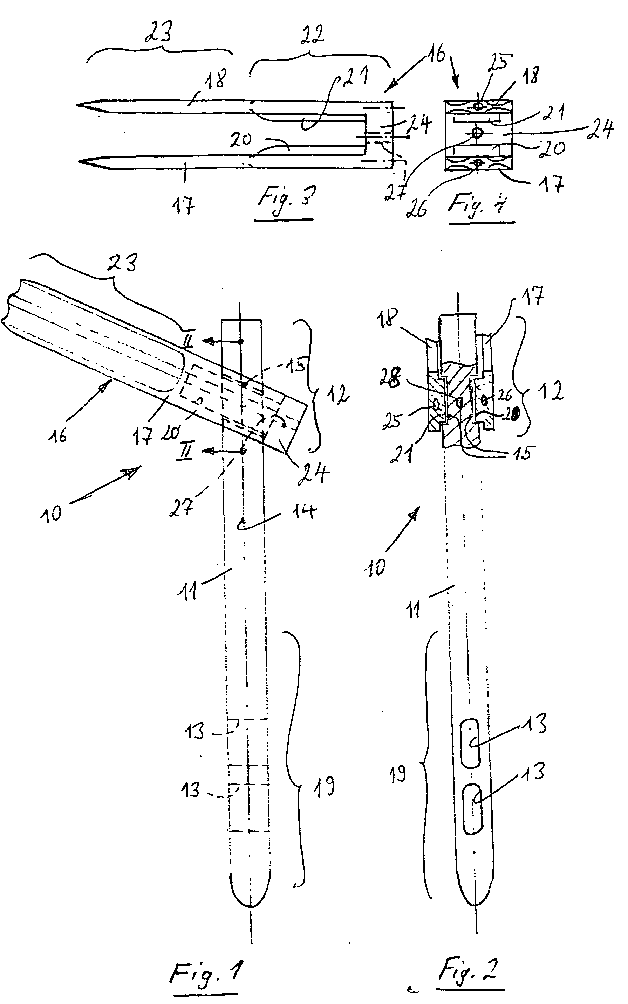

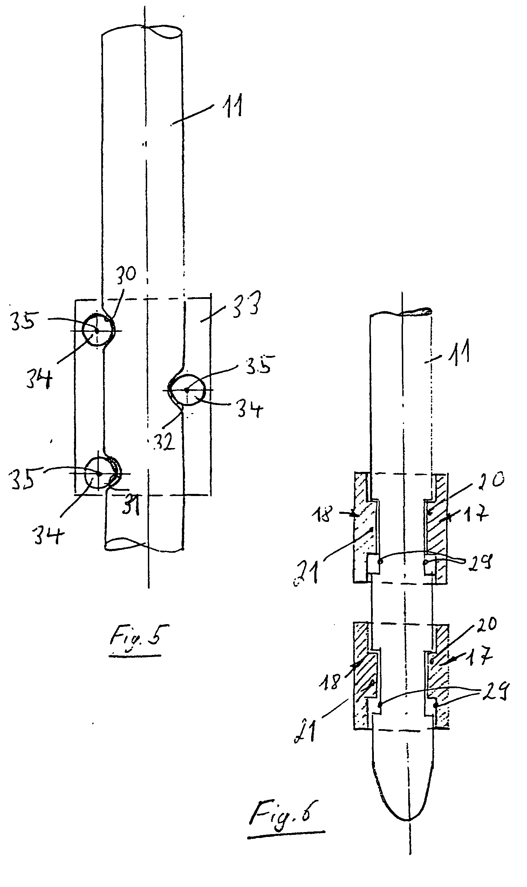

In den Fig. 1 und 2 ist ein Osteosynthese-System 10 zur Versorgung subtrochanterer und pertrochanterer sowie Schenkelhalsfrakturen mit einem von proximal in den Markraum eines Femur einführbaren Marknagel 11, der einen distalen Bereich mit zwei Langloch-Querbohrungen 13 für die Aufnahme eines nicht näher dargestellten distalen Verriegelungsbolzens oder Verriegelungsschraube, sowie einen proximalen Bereich 12 mit sich schräg zur Nagel-Längsachse 14 erstreckenden und relativ zur Längsachse 14 diametral angeordneten Ausnehmungen 15 aufweist, und mit einem von lateral seitlich am Marknagel 11 vorbei in den Schenkelhals und Femurkopf einführbaren Verriegelungselement 16, welches zugleich als Schenkelhalsklinge dient und nach Art einer Stimmgabel mit zwei Zinken 17, 18 ausgebildet ist. Der distale Bereich des Marknagels 12 ist mit der Bezugsziffer 19 gekennzeichnet.

Der Marknagel 11 ist zwischen den beiden Zinken 17, 18 des stimmgabelartigen Verriegelungselements 16 formschlüssig gehalten, und zwar innerhalb der sich schräg erstreckenden Ausnehmungen 15 des Marknagels 11. Zur Plazierung des Verriegelungselements 16 innerhalb der Ausnehmungen 15 sind an den Innenseiten der Zinken 17, 18 Führungsleisten 20, 21 ausgebildet, die im wesentlichen spielfrei in die Ausnehmungen 15 einführbar sind, so wie dies in Fig. 2 dargestellt ist.

Die Führungsleisten 20, 21 definieren einen Kraftträger-Verriegelungsabschnitt 22. Der vom Marknagel 11 wegstehende und in den Knochen eintreibbare Teil der Zinken 17, 18 definiert einen Knochen-Verankerungsabschnitt derselben und ist mit der Bezugsziffer 23 gekennzeichnet. In diesem Bereich sind die Zinken 17, 18 klingenartig ausgebildet, und zwar als I-Profil. Im Bereich des Kraftträger-Verriegelungsabschnitts 22 sind die Zinken 17, 18 ebenfalls profiliert, und zwar komplementär zum Querschnitt der zugeordneten Ausnehmungen 15 des Marknagels 11 zur spielfreien Aufnahme des Verriegelungsabschnitts 22 bzw. des zugeordneten Verriegelungselements 16.

Bei dem dargestellten Ausführungsbeispiel weisen die Ausnehmungen 15 zur Aufnahme der Zinken 17, 18 des Verriegelungselements 16 einen rechteckförmigen Querschnitt auf.

Die Zinken 17, 18 und/oder das stegartige Verbindungselement 24 derselben umfassen eine sich in Zinken-Längsrichtung erstreckende Bohrung 25, 26 bzw. 27 für einen nicht dargestellten Führungs- bzw. Zieldraht. In Fluchtung mit der im Verbindungssteg 24 ausgebildeten Zieldraht-Bohrung 27 ist auch im Marknagel 11 eine Zieldraht-Bohrung 28 ausgebildet. Die vorderen im Femurkopf liegenden Kanten der klingenartigen Zinken 17, 18 sind als meißelartige Schneiden ausgebildet, um das Einschlagen des Verriegelungselements 16 in die Spongiosa des Schenkelhalses und Femurkopfes ohne vorheriges Aufbohren der Spongiosa auf volle Breite der Zinken 17, 18 zu erleichtern. Wie bereits eingangs erwähnt, wird mit der beschriebenen Ausbildung der Zinken 17, 18 mit einem Doppel-T bzw. I-Querschnitt ein extrem hohes Flächenträgheitsmoment und damit extrem hohe Biegesteifigkeit bei minimaler Querschnittsfläche erreicht, so daß beim Einschlagen des Verriegelungselements 16 in den Schenkelhals und Femurkopf nur wenig Spongiosa verdrängt werden muß. Darüber hinaus begünstigt dieser Querschnitt der Zinken 17, 18 die Rotationsstabilität des Femurkopfes und Schenkelhalses im Verhältnis zum Femur bei Versorgung eines Schenkelhalsbruches oder einer pertrochanteren Fraktur.

Wie ebenfalls schon dargelegt, sind die beiden distalen Querbohrungen 13 als Langloch-Querbohrungen ausgebildet, durch die hindurch distale Verriegelungsbolzen oder -schrauben zur Verankerung im Femurknochen durchführbar sind, wobei die Verriegelungsbolzen zur statischen distalen Verriegelung des Marknagels 11 am proximalen Ende (in Fig. 1 und 2 oberen Ende) und zur dynamischen distalen Verriegelung des Marknagels 11 am distalen Ende (in Fig. 1 und 2 unteren Ende) der Langloch-Querbohrungen 13 eingeführt werden. Bei der dargestellten Ausführungsform weisen die Langloch-Querbohrungen 13 jeweils eine axiale Erstreckung bzw. Länge auf, die etwa dem 2,5-fachen Durchmesser der zugeordneten Verriegelungsbolzen bzw. der Breite der Langloch-Querbohrungen 13 entspricht.

Die beiden seitlichen Ausnehmungen 15 des Marknagels 11 für die Aufnahme des stimmgabelartigen Verriegelungselements 16 schließen mit der Längsachse 14 des Marknagels 11 einen Winkel von entweder 125° oder alternativ 135° ein.

Vorzugsweise ist in der durch den Marknagel 11 und das Verriegelungselement 16 definierten Ebene der proximale Abschnitt 12 des Marknagels 11 gegenüber dessen distalem Abschnitt 19 um etwa 6° lateral nach außen gebogen, wobei der Übergang zwischen distalem und proximalem Abschnitt durch einen knickfreien Bogenabschnitt gebildet ist.

Im übrigen weist der Marknagel im vorliegenden Fall einen rotationssymmetrischen bzw. kreisrunden Querschnitt auf. Grundsätzlich ist auch ein ovaler Querschnitt denkbar, um die Rotationsstabilität desselben innerhalb des Femur zusätzlich zu fördern.

Der Marknagel 11 kann durchgehend hohl ausgebildet sein, so daß er über einen nicht näher dargestellten, jedoch an sich bekannten Führungsspieß in den proximalen Femur einführbar ist. Die Wandstärke des hohlen Marknagels 11 beträgt etwa 2,0 mm. Im übrigen ist sowohl der Marknagel 11 als auch das Verriegelungselement 16 aus einem humanverträglichen Material, insbesondere Titan bzw. einer Titanlegierung hergestellt. Gleiches gilt für die distalen Verriegelungsbolzen.

Grundsätzlich ist es auch denkbar, daß der intrameduläre Kraftträger bzw. hier Marknagel 11 am proximalen und/oder distalen Endbereich zur formschlüssigen Anlage wenigstens einer an der dem Kraftträger zugewandten Seite abgeflachten Zinke des Verriegelungselements 16 wenigstens an der dieser Zinke zugewandten Seite entsprechend abgeflacht ist. Auch durch diese einfache Maßnahme ist eine rotationsstabile Verankerung des Marknagels 11 möglich, vor allem im distalen Bereich desselben.

In Fig. 6 ist eine distale Verriegelung eines Marknagels 11 mittels eines Verriegelungselements entsprechend dem Verriegelungselement 16 gemäß den Fig. 3 und 4 dargestellt. Von besonderer Bedeutung ist, daß die den Zinken 17, 18 zugeordneten Ausnehmungen 29 in Längsrichtung des Marknagels 11 verlängert sind, so daß sowohl eine statische als auch dynamische Verriegelung des Marknagels 11 mittels eines Verriegelungselements der vorbeschriebenen Art möglich ist, wobei die Verriegelungselemente zur statischen distalen Verriegelung des Marknagels 11 am proximalen Ende (in Fig. 6 oberen Ende) und zur dynamischen distalen Verriegelung des Marknagels 11 am distalen Ende (in Fig. 6 unteren Ende) der Ausnehmungen 29 anliegen, wobei die Anlage durch die in die Ausnehmungen 29 eingepaßten Führungsleisten 20, 21 erfolgt. Die Breite der Führungsleisten 20, 21 ist also geringer als die Breite der Ausnehmungen 29. Dann ist sowohl eine statische als auch dynamische Verriegelung in der beschriebenen Weise möglich.

In Fig. 5 ist eine alternative Ausführungsform eines erfindungsgemäßen Nagelsystems dargestellt. Am proximalen und/oder distalen Ende eines Marknagels 11 sind an einer Seite zwei halbkreisförmige Ausnehmungen im Längsabstand voneinander ausgebildet. An der gegenüberliegenden Seite ist eine dritte halbkreisförmige Ausnehmung vorgesehen, und zwar auf etwa halbem Abstand zwischen den beiden erstgenannten Ausnehmungen. Die erwähnten Ausnehmungen sind mit den Bezugsziffern 30, 31 und 32 gekennzeichnet.

In diese Ausnehmungen 30, 31 und 32 sind drei entsprechend angeordnete und durch eine Verbindungsplatte 33 starr miteinander verbundene Zinken 34 einführbar, so daß eine spielfreie Verriegelung zwischen Marknagel 11 und dem durch die Zinken 34 samt Verbindungsplatte 33 gebildeten Verriegelungselement erhalten wird. Die Zinken 34 haben einen kreisrunden Querschnitt. Die freien Enden der Zinken 34 sind als Spitzen 35 ausgebildet. Auf diese Weise lassen sich die Zinken 34 relativ leicht in Spongiosa ohne vorherige Aufbohrung derselben einschlagen.

Die Anzahl der Ausnehmungen 30, 31, 32 kann beliebig gewählt werden. In der Regel werden jedoch mehr als drei Zinken entsprechend Fig. 5 nicht benötigt. Für die meisten Fälle genügt ein Verriegelungselement mit nur zwei Zinken, d.h. ein nach Art einer Stimmgabel ausgebildetes Verriegelungselement entsprechend den Fig. 1 bis 4 bzw. 6.

### Bezugszeichenliste

- 10: Osteosynthese-System
- 11: Marknagel
- 12: proximaler Bereich
- 13: Langloch-Querbohrung
- 14: Nagel-Längsachse
- 15: Ausnehmung
- 16: Verriegelungselement
- 17: Zinke
- 18: Zinke
- 19: distaler Bereich
- 20: Führungsleiste
- 21: Führungsleiste
- 22: Kraftträger-Verriegelungsabschnitt
- 23: Knochen-Verankerungsabschnitt
- 24: Verbindungselement
- 25: Bohrung
- 26: Bohrung
- 27: Bohrung
- 28: Zieldraht-Bohrung
- 29: Ausnehmung
- 30: Ausnehmung
- 31: Ausnehmung
- 32: Ausnehmung
- 33: Verbindungsplatte
- 34: Zinken

## Patentansprüche

1. System bestehend aus einem intramedulären Kraftträger, insbesondere Marknagel (11), und wenigstens einem diesem zugeordneten Knochen-Verriegelungselement (16),
**dadurch gekennzeichnet, daß**
das Verriegelungselement (16) wenigstens zwei Zinken (17, 18) aufweist, zwischen denen der Kraftträger (11) form- und/oder kraftschlüssig gehalten sein kann.

2. System nach Anspruch 1,
**dadurch gekennzeichnet, daß**
der Kraftträger (11) am proximalen und/oder distalen Ende wenigstens eine sich quer zur Längsachse (14) erstreckende Ausnehmung (15 bzw. 29) zur formschlüssigen Aufnahme wenigstens einer Zinke (17, 18) des Verriegelungselements (16) aufweist.

3. System nach Anspruch 1,
**dadurch gekennzeichnet, daß**
der Kraftträger (11) am proximalen und/oder distalen Ende zur formschlüssigen Anlage wenigstens einer an der dem Kraftträger (11) zugewandten Seite abgeflachten Zinke (17 und/oder 18) des Verriegelungselements (16) wenigstens an der dieser Zinke zugewandten Seite entsprechend abgeflacht ist.

4. System nach Anspruch 1 oder 2,
**dadurch gekennzeichnet, daß**
die Zinken (17, 18) einen Kraftträger-Verriegelungs (22) - und einen Knochen-Verankerungs (23) -Abschnitt aufweisen, wobei im Bereich des ersteren die Zinken an der dem Kraftträger (11) zugewandten Seite profiliert sind (Führungsleisten 20, 21) entsprechend einer komplementären Profilierung der den Zinken (17, 18) zugeordneten und zur spielfreien Aufnahme derselben dienenden Ausnehmungen (15 bzw. 29) des Kraftträgers (11).

5. System nach einem der Ansprüche 1 bis 4,
**dadurch gekennzeichnet, daß**
das Verriegelungselement (16) drei oder mehr in Kraftträger-Längsrichtung jeweils versetzt zueinander angeordnete Zinken (34) umfaßt.

6. System nach einem der Ansprüche 1 bis 5,
**dadurch gekennzeichnet, daß**
die Zinken (17, 18) im Bereich ihrer Knochen-Verankerungsabschnitte (23) entweder jeweils nach Art einer Klinge mit I-, T-, Rechteck- oder Oval-Querschnitt, oder stiftartig mit Stern-, Dreieck- oder Kreis-Querschnitt ausgebildet sind.

7. System nach einem der Ansprüche 1 bis 5,
**dadurch gekennzeichnet, daß**
das Verriegelungselement (16) stimmgabelartig ausgebildet ist.

8. System nach einem der Ansprüche 1 bis 7,
**dadurch gekennzeichnet, daß**
die Zinken (17, 18) und/oder das Verbindungselement (24 bzw. 33) derselben eine sich in Zinken-Längsrichtung erstreckende Bohrung (25, 26 bzw. 27) für einen Führungs- bzw. Zieldraht umfassen.

9. System nach einem der Ansprüche 2 und 4 bis 8,
**dadurch gekennzeichnet, daß**
die Ausnehmungen (29) zur Aufnahme wenigstens einer Zinke (17, 18) des Verriegelungselements (16) am proximalen oder distalen Endbereich des Kraftträgers (11) in Längsrichtung desselben verlängert sind, so daß sowohl eine statische als auch dynamische proximale oder distale Verriegelung des Kraftträgers (11) möglich ist.

10. System nach einem der Ansprüche 2 und 4 bis 9,
**dadurch gekennzeichnet, daß**
die Ausnehmungen (15) zur Aufnahme wenigstens einer Zinke (17, 18) des Verriegelungselements (16) sich schräg zur Längsachse (14) des Kraftträgers (11) erstreckt.

## Claims

1. System consisting of an intramedullary load-bearing member, especially a medullary nail (11), and at least one bone-locking element (16) associated therewith,
**characterized in that**
the locking element (16) has at least two tines (17, 18) between which the load-bearing member (11) can be held by interlocking and/or friction.

2. System according to claim 1,
**characterized in that**
the load-bearing member (11) has, at the proximal and/or distal end, at least one recess (15 and/or 29) that extends transversely relative to the longitudinal axis (14) for the interlocked reception of at least one tine (17, 18) of the locking element (16).

3. System according to claim 1,
**characterized in that**
the load-bearing member (11), for interlocking contact with at least one tine (17 and/or 18) of the locking element (16) that is flattened on the side facing the load-bearing member (11), is, at the proximal and/or distal end, correspondingly flattened at least on the side facing the tine in question.

4. System according to claim 1 or 2,
**characterized in that**
the tines (17, 18) have a load-bearing member-locking portion (22) and a bone-anchoring portion (23), wherein in the region of the former the tines are shaped on the side facing the load-bearing member (11) (guide ribs 20, 21) in correspondence with complementary shaping of the recesses (15 and/or 29) of the load-bearing member (11) that are associated with the tines (17, 18) and serve to receive the tines without play.

5. System according to any one of claims 1 to 4,
**characterized in that**
the locking element (16) comprises three or more tines (34) arranged offset relative to one another in the longitudinal direction of the load-bearing member.

6. System according to any one of claims 1 to 5,
**characterized in that**
the tines (17, 18), in the region of their bone-anchoring portions (23), are each either blade-like with an I-shaped, T-shaped, rectangular or oval cross-section or are pin-like with a star-shaped, triangular or circular cross-section.

7. System according to any one of claims 1 to 5,
**characterized in that**
the locking element (16) has a tuning-fork-like form.

8. System according to any one of claims 1 to 7,
**characterized in that**
the tines (17, 18) and/or the connecting element (24; 33) thereof comprise a bore (25, 26 and/or 27) for a guiding or pilot wire, which bore extends in the longitudinal direction of the tines.

9. System according to any one of claims 2 and 4 to 8,
**characterized in that**
the recesses (29) for receiving at least one tine (17, 18) of the locking element (16) at the proximal or distal end region of the load-bearing member (11) are extended in the longitudinal direction thereof, so that either static or dynamic proximal or distal locking of the load-bearing member (11) is possible.

10. System according to any one of claims 2 and 4 to 9,
**characterized in that**
the recesses (15) for receiving at least one tine (17, 18) of the locking element (16) extends obliquely relative to the longitudinal axis (14) of the load-bearing member (11).

## Revendications

1. Système composé d'un clou intramédullaire, en particulier d'une broche (11), et d'au moins un élément d'accrochage à l'os (16) attribué à celui-ci,
**caractérisé en ce que** l'élément d'accrochage (16) comporte au moins deux pointes (17, 18) entre lesquelles le clou (11) peut être maintenu par coopération de formes et/ou par la force.

2. Système selon la revendication 1, **caractérisé en ce que** le clou (11) comprend, sur l'extrémité proximale et/ou distale, au moins un creux (15 ou 29), s'étendant transversalement à l'axe longitudinal (14), pour le logement par coopération de forme d'au moins une pointe (17, 18) de l'élément d'accrochage (16).

3. Système selon la revendication 1, **caractérisé en ce que**, sur l'extrémité proximale et/ou distale, pour l'agencement, par coopération de formes d'au moins une pointe (17 et/ou 18) aplatie du côté tourné vers le clou (11) de l'élément d'accrochage (16), le clou (11) est aplati de manière correspondante au moins sur le côté tourné vers cette pointe.

4. Système selon la revendication 1 ou 2, **caractérisé en ce que** les pointes (17, 18) comportent une partie d'accrochage du clou (22) et une partie de blocage à l'os (23), au niveau de la première, les pointes étant profilées (baguettes de guidage 20, 21) sur le côté tourné vers le clou (11) conformément à un profil complémentaire des creux (15 ou 29) du clou (11) attribués aux pointes (17, 18) et servant au logement sans jeu des ces mêmes pointes.

5. Système selon l'une quelconque des revendications 1 à 4, **caractérisé en ce que** l'élément d'accrochage (16) comprend trois pointes (34) ou plus disposées de manière décalée les unes par rapport aux autres dans le sens longitudinal du clou.

6. Système selon l'une quelconque des revendications 1 à 5, **caractérisé en ce que** les pointes (17, 18) sont réalisées, au niveau de leurs parties de blocage à l'os (23), soit sous forme de lame avec une section en I, T, rectangulaire ou ovale ou sous forme de goupille avec une section en étoile, triangulaire ou circulaire.

7. Système selon l'une quelconque des revendications 1 à 5, **caractérisé en ce que** l'élément d'accrochage (16) est réalisé sous forme de diapason.

8. Système selon l'une quelconque des revendications 1 à 7, **caractérisé en ce que** les pointes (17, 18) et/ou l'élément de connexion (24 ou 33) de celles-ci comprennent un trou (25, 26 ou 27) s'étendant dans le sens longitudinal des pointes pour un fil de guidage ou de repérage.

9. Système selon l'une quelconque des revendications 2 et 4 à 8, **caractérisé en ce que** les creux (29), pour le logement d'au moins une pointe (17, 18) de l'élément d'accrochage (16) sont prolongés sur la zone d'extrémité proximale ou distale du clou (11) dans le sens longitudinal de celui-ci de telle sorte qu'à la fois un accrochage statique et dynamique proximal ou distal du clou (11) soit possible.

10. Système selon l'une quelconque des revendications 2 et 4 à 9, **caractérisé en ce que** les creux (15), pour le logement d'au moins une pointe (17, 18) de l'élément d'accrochage (16) s'étend en biais par rapport à l'axe longitudinal (14) du clou (11).
